# EUROPEAN PATENT SPECIFICATION

(11) **EP 3 998 039 B1**
(45) Date of publication and mention of the grant of the patent: **19.06.2024**
(21) Application number: 20911305.9
(22) Date of filing: 27.11.2020
(51) Int. Cl.: A61B 1/00, A61B 90/50, A61B 90/16, A61B 90/57, A61B 1/24, A61B 13/00

(54) **MOUTH SPECULUM SUPPORT DEVICE**
HALTEVORRICHTUNG FÜR EIN MUNDSPEKULUM
DISPOSITIF DE SUPPORT DE SPÉCULUM BUCCAL

(30) Priority: 17.09.2020 CN 202022045548 U
(43) Date of publication of application: 18.05.2022
(73) Proprietor: FUJIAN MEDICAL UNIVERSITY UNION HOSPITAL, Fuzhou, Fujian 350001 (CN); HUANG, Jianmin, Fuzhou, Fujian 350001 (CN)
(72) Inventor: HUANG, Jianmin, Fuzhou, Fujian 350001 (CN); YANG, Shiming, Fuzhou, Fujian 350001 (CN)
(74) Representative: Bayramoglu et al.
(86) International application number: PCT/CN2020/132236
(87) International publication number: WO 2022/057090

(56) References cited:
- WO-A1-2020/136445
- CN-A- 107 049 218
- CN-A- 111 035 354
- CN-U- 207 590 651
- CN-U- 208 910 189
- CN-U- 209 826 647
- CN-U- 210 019 273
- CN-U- 211 155 690
- CN-U- 211 324 880
- DE-U1- 29 622 630
- US-A1- 2007 213 597
- US-A1- 2012 238 828
- US-A1- 2015 087 918

## Description

### Technical field

The present invention relates to a mouth gag supporting device.

### Background Technology

During the general anesthesia of pharynx and oral cavity in clinic, mouth gag should be used to keep the mouth open. In order to fully expose the deep part of mouth and pharynx cavity, the assistant should continuously lift the handle of tongue depressor during the operation, this process not only consumes manpower, but also takes up space, or the handle of tongue depressor should be padded up with instruments such as bowls and plates, but this method is unstable and easy to slip off, therefore, according to the principle of hook suspension, a variety of existing mouth gap supporting devices are designed, but the existing mouth gap supporting devices are rough and simple in structure, inconvenient in use, unable to accurately adjust the angle and strength during the operation, and prone to tissue damage.

US 2015/087918 A1 discloses a surgical retractor system and method, it describes a multiple piece tongue blade, the multiple piece tongue blade is provided with a step and a tip. The stem is adapted to be inserted into a handle of a surgical retractor. The tip is adapted to be selectively attached to the stem. The tip is insertable into a mouth of a patient such that the tip applies pressure onto a tongue of the patient.

CN 211324880U discloses a mouth gag support, the mouth gag support comprises a base, a supporting column, an adjusting arm and a mouth gag connector. The lower end of the supporting column is installed on the base, the near end of the adjustingarm is provided with a positioning adjusting hole, and the far end is provided with a mouth gag connector. The adjusting arm is connected to the supporting column through the positioning adjusting hole, when the adjusting arm is in a loose state, the adjusting arm can slide up and down along the supporting column, and therefore the angle and the opening degree of the mouth gag connected to the adjusting arm are adjusted. According to the mouth gag supporting frame, the locking or loosening state of the adjusting arm can be switched by simply lifting or pressing the adjusting arm, so that the position of the adjusting arm on the supporting column is conveniently adjusted, the purpose of adjusting the mouth gag is achieved, and the clinical use process is very simple.

CN 210019273U discloses an oral cavity drag hook system. The retractor comprises an oral cavity retractor device, a soft palate retractor device and a pharyngeal posterior wall soft tissue distraction device, the oral cavity retractor device comprises a tongue pressing assembly, a first adjusting part and an upper tooth distraction assembly, the tongue pressing assembly and the upper tooth distraction assembly are both connected with the first adjusting part, and the first adjusting part adjusts the tongue pressing assembly and the upper tooth distraction assembly to distraction a lower jaw and an upper jaw; the pharyngeal posterior wall soft tissue distraction device is located above the oral cavity drag hook device, the pharyngeal posterior wall soft tissue distraction device comprises two soft tissue distraction assemblies and a second adjusting part connected with the two soft tissue distraction assemblies, and the second adjusting part adjusts the two soft tissue distraction assemblies to distraction pharyngeal posterior wall soft tissue; the soft palate retractor device is located between the two soft tissue distraction assemblies, the oral cavity is easily distraction through the oral cavity retractor system, the rear wall of the oral pharynx is shown in front of an operator to the maximum extent, a good and stable operation visual field is kept, and the requirements of the operator are met.

US 2007/213597 A1 discloses a surgical clamp for use with a surgical retractor support frame. The surgical clamp has a handle that rotates about a first pivot point from a first position to a second position, and a second pivot point eccentric to the first pivot point. The surgical clamp also has an intermediate link with a first portion that rotates about the second pivot point and a second portion that is rotatably connected to a third pivot point on an output link. The intermediate link is manipulated by rotation of the handle, and the intermediate link further manipulates the output link from a first position to a second position. The output link is operably connected to at least one of the first and second clamps to apply a clamping force on a surgical instrument to lock the surgical instrument in place.

US 2012/238828 A1 discloses a surgical retractor fixing device, having a clamp body arranged for removably fixing the surgical retractor fixing device to a frameset arranged to be mounted on an operating table; a rotating body attached to the clamp body, the rotating body pivotable around an axis of rotation and having a fixing element arranged for fixing a surgical retractor; the clamp body having a mouth to at least partially encompass the frameset and the clamp body comprising a locking device, the locking device having a locking rod arranged for engaging with the frameset thus fixing the clamp body to the frameset and a locking lever arranged for actuating the locking rod, the locking lever being pivotable around a locking lever swivel axis, the locking rod being arranged to exert a rotating body clamping force on the rotating body so that the rotating body is clamped when the locking lever actuates the locking rod.

### Content of the Invention

The present invention provides a mouth gap supporting device to solve the problem that the existing opening device is inconvenient to use, the angle and force cannot be accurately adjusted during the operation, and the tissue damage is easy to occur.

The solution to the technical problem of the present invention is: a mouth gag supporting device, characterized in that, it includes a gripping structure and an eccentric shaft structure, said gripping structure is connected to the mouth gag, said eccentric shaft structure is composed of an eccentric structure and a shaft structure, the eccentric structure is fixed to the gripping structure, the eccentric structure is provided with a U-shaped slot, the end of the shaft structure is eccentrically hinged in the U-shaped slot, and the eccentric shaft structure is locked by a fastening nut;
said gripping structure is L-shaped, with the horizontal part at the bottom and the vertical part at the top, and the horizontal part is provided with a C-shaped slot for connecting the mouth gag;
eccentric holes are relatively arranged on the two parallel circular arc feet of the U-shaped slot;
said shaft structure includes a stud and an extension sleeve, one end of the stud is rotatably connected between the eccentric holes on the two parallel circular arc feet by an eccentric bolt, the other end of the stud is fixed to the extension sleeve;
said stud is screwed with the fastening nut.

Further, the end of the vertical part of the gripping structure is integrally formed with the eccentric structure.

Further, the end of the vertical part of the gripping structure is fixed with the eccentric structure, and the connection part of the U-shaped slot connecting the two parallel circular arc feet is fixed to the vertical part of the gripping structure through a latch.

Further, the side wall of the vertical part of the gripping structure is provided with an adjustment screw for adjusting the movement of the latch.

Further, an extension rod is fixed in the extension sleeve by a bolt.

Further, one end of the extension rod is fixed in the extension sleeve and the other end is provided with a stable supporting structure.

Compared with the prior art, the present invention has the following beneficial effects: light structure, easy installation, stable support, infinitely fine adjustment of the support angle and strength within ±90°, and stable and effective exposure of the oral cavity and pharyngeal cavity during the operation.

### Illustration

The following is a further description of the present invention in combination with the drawings.
FIG. 1 is a schematic diagram of the structure of the present invention embodiment 1.
FIG. 2 is an exploded view of the present invention embodiment 1.
FIG. 3 is a schematic diagram of the structure of the extension rod.
FIG. 4 is a schematic diagram of the structure of the present invention embodiment 2.
FIG. 5 is an exploded view of the present invention embodiment 2.
FIG. 6 is a schematic diagram of the structure of the C-shaped slot located at the side of the horizontal part.
FIG. 7 is a diagram of the present invention in use.

In the figure: 1-mouth gag supporting device; 2-existing mouth gag with tongue depressor; 3-gripping structure; 4-U-shaped slot; 5-stud; 6-fastening nut; 7-extension sleeve; 8-bolt; 9-C-shaped slot; 10-eccentric hole; 11-eccentric bolt; 12-light hole; 13-bolt; 14-horizontal seat plate; 15-latch; 16-vertical jack; 17-adjusting screw; 19-extension rod; 20-stable supporting structure.

### Specific Embodiments

The present invention will be further described in combination with the drawings and specific embodiments.

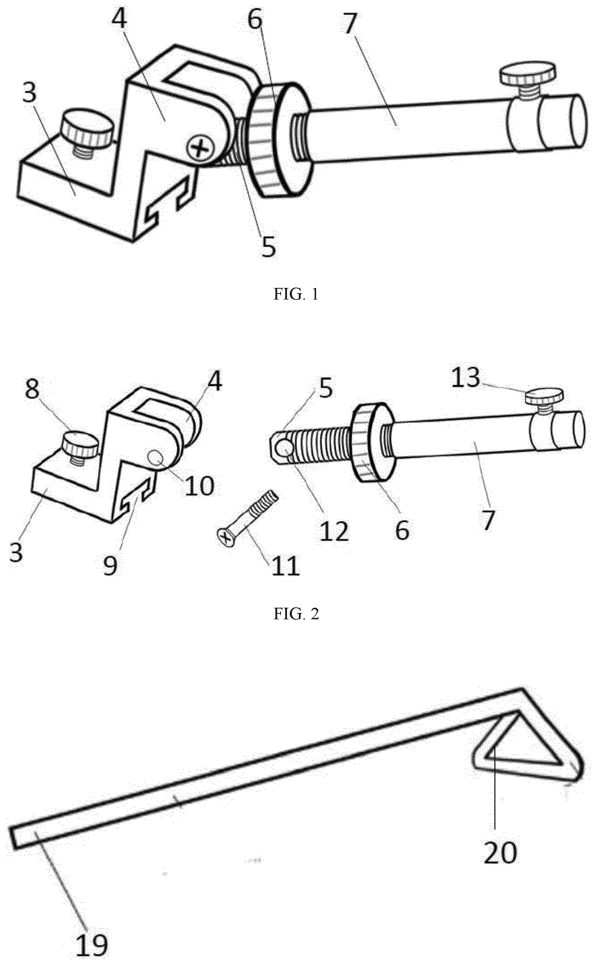

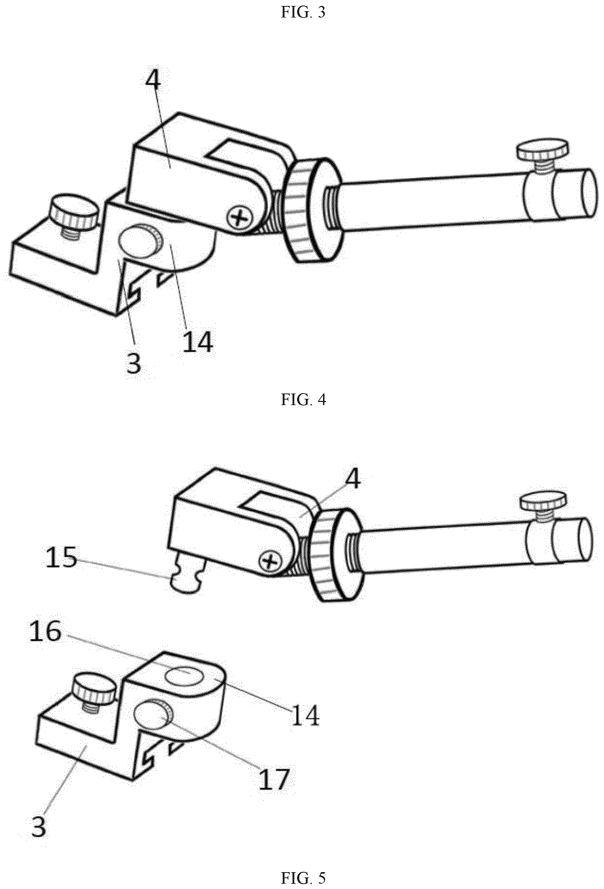

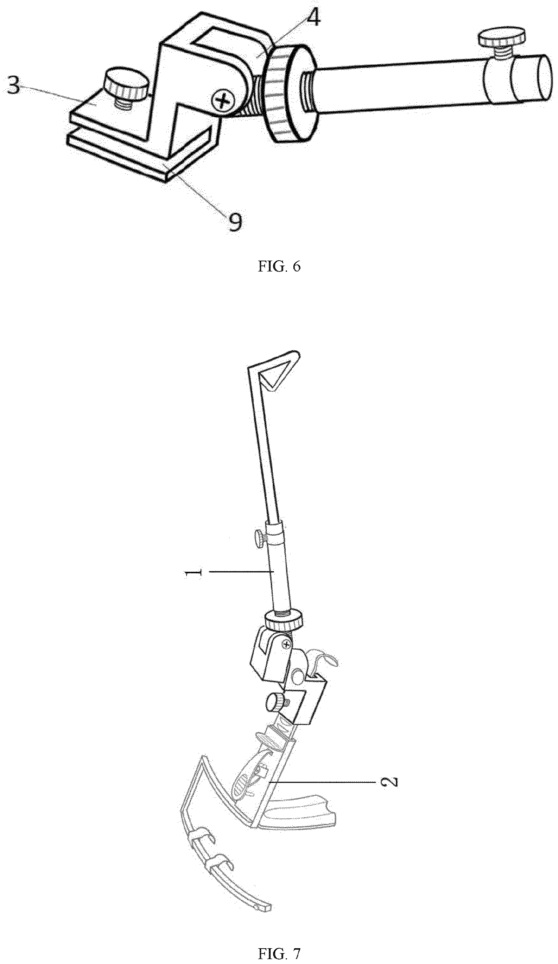

As shown in FIG. 1-7, embodiment 1, a mouth gag supporting device 1, includes a gripping structure 3 and an eccentric shaft structure, said gripping structure is connected to the mouth gag, said eccentric shaft structure is composed of an eccentric structure and a shaft structure, the eccentric structure is fixed to the gripping structure, the eccentric structure is provided with a U-shaped slot 4, the end of the shaft structure is eccentrically hinged in the U-shaped slot and the eccentric shaft structure is locked by a fastening nut.

In this embodiment, said gripping structure is L-shaped, the horizontal part is at the bottom, the vertical part is at the top, the horizontal part is provided with a C-shaped slot 9 for connecting the mouth gag 2, the C-shaped slot can be set at the bottom of the horizontal part or the side of the horizontal part, and then the top surface of the horizontal part is penetrated with a bolt 8 for locking the mouth gag in the C-shaped slot.

In this embodiment, eccentric holes 10 are relatively opened on the two parallel circular arc feet of the U-shaped slot, and the ends of the two parallel circular arc feet of the U-shaped slot are circular arc shape, and the eccentric holes are not coincident with the circular position of the circular arc of the ends of the two parallel circular arc feet of the U-shaped slot, and the eccentric holes can only be located below the circular position.

In this embodiment, said shaft structure includes stud 5, extension sleeve 7, one end of the stud is rotatably connected between the eccentric holes on the two parallel circular arc feet by eccentric bolts, the other end of the stud is fixed to the extension sleeve or the other end of the stud is directly integrated into the extension sleeve, an extension rod 19 is fixed inside the extension sleeve by bolts 13; one end of the stud is rotatably connected between the eccentric holes on the two parallel circular arc feet by eccentric bolts 11, and the end of the stud is provided with a light hole 12 for the eccentric bolt to pass through, and the eccentric bolt is a bolt with threads set on the side away from the bolt head and a light shaft on the side near the bolt head, and the length of the light shaft is equal to the distance between the two parallel circular arc feet of the U-shaped slot, and the eccentric bolt passes through the eccentric hole on one side of the parallel circular arc feet, the light hole, and the eccentric hole on the other side of the parallel circular arc feet in turn, then the stud is rotatably connected to the light shaft through nut locking.

In this embodiment, said stud is screwed with a fastening nut 6; by adjusting the fastening nut fixed on the end arc of the parallel circular arc foot (tangent) at a certain position, the shaft is positively locked at the end point of the eccentric radius, during the operation, open the mouth with the mouth gap with the tongue depressor, lift the handle of the tongue depressor to the desired angle and strength, fastening the nut so that the shaft is fixed, that is, to produce a stable support effect. The eccentric shaft can be adjusted by the tightening nut to achieve an infinitely variable diameter, producing a lifting support effect at any angle within ±90°.

In this embodiment, one end of said extension rod is fixed in the extension sleeve by screws, and the other end is provided with a stable supporting structure 20, the extension rod is a solid metal rod, diameter 7.0mm ~ 8.0mm, length 300mm ~ 400mm, the stable supporting structure is a triangular or circular structure, through the stable supporting structure seat against the instrument table to make the support stable.

In this embodiment, the vertical part of the gripping structure is integrally shaped with the eccentric structure on the outside of the end of the vertical part.

The above-mentioned embodiment is a one-piece gripping structure, and a second combined gripping structure is provided, embodiment 2, because the other structures are the same as embodiment 1, so it is not repeated here, the vertical part of said gripping structure has an eccentric structure inserted on the end of the vertical part, the eccentric structure has eccentric holes on the two parallel circular arc feet of the U-shaped slot, and the lower end face of the connection part of the U-shaped slot connecting the two parallel circular arc feet is fixed to the vertical part of the gripping structure by a latch.

The gripping structure is L-shaped, with the horizontal part at the bottom and the vertical part at the top, and the vertical part is provided with a horizontal seat plate 14, and the horizontal seat plate is provided with a vertical jack 16, and the lower face of the connecting part on the U-shaped slot is provided with a latch 15, and one end of the latch is connected to the connecting part on the U-shaped slot and the other end is inserted into the vertical jack.

In this embodiment, the side wall of the horizontal seat plate of the vertical part of the said gripping structure is provided with an adjustment screw 17 for adjusting the movement of the latch for locking the latch.

In this embodiment, the combined gripping structure can achieve horizontal movement of the support bar and stable support when the head position of the surgical patient deviates from the midline.

Unless otherwise stated, if any of the above technical solutions disclosed in the present invention discloses a range of values, the disclosed range of values is the preferred range of values, and any person skilled in the art should understand that the preferred range of values is only the more obvious or representative value of technical effect among many implementable values. Because of the large number of values, it is impossible to enumerate them exhaustively, so the present invention only discloses some of the values to illustrate the technical solution of the present invention, and the above listed values should not constitute a limitation of the scope of protection of the present invention creation.

If the words "first" and "second" are used herein to qualify the parts, a person skilled in the art should know that the use of "first" and "second" is only for the purpose of describing the parts to distinguish them, if not stated otherwise, the above words have no special meaning.

If the present invention discloses or relates to parts or structural members that are fixedly connected to each other, then, unless otherwise stated, a fixed connection can be understood as a fixed connection that can be disassembled (e.g., using bolts or screws) or as a fixed connection that is not disassembled (e.g., riveted, welded), but, of course, a fixed connection can also be replaced by a one-piece structure (e.g., made in one piece using a casting process) (except where a one-piece forming process is clearly not possible).

"down", "front", "back", "left", "right ", "vertical", "horizontal", "top", "bottom", "bottom "inside", "outside" and the like indicate orientation or positional relationships based on the orientation or positional relationships shown in the accompanying drawings, and are intended only to facilitate the description of the present patent, and do not indicate or imply that the device or element referred to must have a particular orientation, be constructed in a particular orientation and The terms used to denote shape in any of the above technical solutions disclosed in the present invention shall, unless otherwise stated, have a meaning that includes a shape that is similar, analogous or close thereto.

Any of the components provided by the present invention may be assembled from a plurality of individual components or may be a separate component manufactured by a one-piece forming process.

Finally, it should be noted that the above examples are only used to illustrate the technical solution of the present invention, not to limit it; although the present invention has been described in detail with reference to the preferred embodiment, a person of ordinary skill in the field should understand that it is still possible to modify the specific implementation of the present invention or to replace some technical features with equivalent ones. The invention is defined in appended independent claim 1. Further embodiments are defined in appended dependent claims.

## Claims

1. A mouth gag supporting device that includes a gripping structure (3) and an eccentric shaft structure, said gripping structure (3) is connected to the mouth gag, said eccentric shaft structure is composed of an eccentric structure and a shaft structure, the eccentric structure is fixed to the gripping structure (3), the eccentric structure is provided with a U-shaped slot (4), the end of the shaft structure is eccentrically hinged in the U-shaped slot, and the eccentric shaft structure is locked by a fastening nut (6) to the eccentric structure;
said gripping structure (3) is L-shaped, with the horizontal part at the bottom and the vertical part at the top, and the horizontal part is provided with a C-shaped slot (9) for connecting the mouth gag (2);
eccentric holes (10) are relatively arranged on the two parallel circular arc feet of the U-shaped slot (4);
said shaft structure includes a stud (5) and an extension sleeve (7), one end of the stud (5) is rotatably connected between the eccentric holes (10) on the two parallel circular arc feet by an eccentric bolt (11), the other end of the stud (5) is fixed to the extension sleeve (7);
said stud (5) is screwed with the fastening nut (6).

2. The mouth gag supporting device according to claim 1, **characterized in that**, the end of the vertical part of the gripping structure is integrally formed with the eccentric structure.

3. The mouth gag supporting device according to claim 1, **characterized in that**, the end of the vertical part of the gripping structure is fixed with the eccentric structure, and the connection part of the U-shaped slot (4) connecting the two parallel circular arc feet is fixed to the vertical part of the gripping structure through a latch (15).

4. The mouth gag supporting device according to claim 3, **characterized in that**, the side wall of the vertical part of the gripping structure is provided with an adjustment screw (17) for adjusting the movement of the latch (15).

5. The mouth gag supporting device according to claim 2 or 4, **characterized in that**, an extension rod (19) is fixed in the extension sleeve (7) by a bolt (13).

6. The mouth gag supporting device according to claim 5, **characterized in that**, one end of the extension rod (19) is fixed in the extension sleeve and the other end is provided with a stable supporting structure (20).

## Patentansprüche

1. Haltevorrichtung für einen Mundsperrer, der eine Greifstruktur (3) und eine exzentrischen Wellenstruktur aufweist, wobei die Greifstruktur (3) mit dem Mundsperrer verbunden ist, die exzentrische Wellenstruktur aus einer exzentrischen Struktur und einer Wellenstruktur zusammengesetzt ist, die exzentrische Struktur ist befestigt an der Greifstruktur (3), die exzentrische Struktur ist mit einem U-förmigen Schlitz (4) versehen, das Ende der Wellenstruktur ist exzentrisch in dem U-förmigen Schlitz schwenkbar angebracht, und die exzentrische Wellenstruktur ist mit einer Befestigungsmutter (6) an der exzentrischen Struktur verriegelt;
die Greifstruktur (3) ist L-förmig, mit einem horizontalen Teil unten und einem vertikalen Teil oben, und der horizontale Teil ist mit einem C-förmigen Schlitz (9) zum Verbinden der Mundsperre (2) versehen;
exzentrische Löcher (10) sind an den beiden parallelen kreisbogenförmigen Füßen des U-förmigen Schlitzes (4) relativ zueinander angeordnet
die Wellenstruktur beinhaltet einen Stift (5) und eine Verlängerungshülse (7), wobei ein Ende des Stiftes (5) zwischen den exzentrischen Löchern (10) auf den beiden parallelen kreisbogenförmigen Füßen durch einen exzentrischen Bolzen (11) drehbar verbunden ist, das andere Ende des Stiftes (5) an der Verlängerungshülse (7) befestigt ist;
der Stift (5) mit der Befestigungsmutter (6) verschraubt wird.

2. Haltevorrichtung für einen Mundsperrer nach Anspruch 1, **dadurch gekennzeichnet, dass** das Ende des vertikalen Teils der Greifstruktur einstückig mit der exzentrischen Struktur ausgebildet ist.

3. Haltevorrichtung für einen Mundsperrer nach Anspruch 1, **dadurch gekennzeichnet, dass** das Ende des vertikalen Teils der Greifstruktur an der exzentrischen Struktur befestigt ist und dass der Verbindungsteil des U-förmigen Schlitzes (4), der die beiden parallelen kreisbogenförmigen Füße verbindet, durch einen Riegel (15) an dem vertikalen Teil der Greifstruktur befestigt ist.

4. Haltevorrichtung für einen Mundsperrer nach Anspruch 3, **dadurch gekennzeichnet, dass** an der Seitenwand des vertikalen Teils der Greifstruktur eine Einstellschraube (17) zum Einstellen der Bewegung des Riegels (15) vorgesehen ist.

5. Haltevorrichtung für einen Mundsperrer nach Anspruch 2 oder 4, **dadurch gekennzeichnet, dass** eine Verlängerungsstange (19) in der Verlängerungshülse (7) durch einen Bolzen (13) befestigt ist.

6. Haltevorrichtung für einen Mundsperrer nach Anspruch 5, **dadurch gekennzeichnet, dass** ein Ende der Verlängerungsstange (19) in der Verlängerungshülse befestigt ist und am anderen Ende eine stabile Stützstruktur (20) vorgesehen ist.

## Revendications

1. Dispositif de support d'ouvre-bouche qui comprend une structure de préhension (3) et une structure d'arbre excentrique, ladite structure de préhension (3) est reliée à l'ouvre-bouche, ladite structure d'arbre excentrique est composée d'une structure excentrique et d'une structure d'arbre, la structure excentrique est fixée à la structure de préhension (3), la structure excentrique est pourvue d'une fente en forme de U (4), l'extrémité de la structure d'arbre est articulée de manière excentrique dans la fente en forme de U, et la structure d'arbre excentrique est verrouillée par un écrou de fixation (6) à la structure excenrique ;
ladite structure de préhension (3) est en forme de L, avec la partie horizontale en bas et la partie verticale en haut, et la partie horizontale est pourvue d'une fente en forme de C (9) pour relier l'ouvre-bouche (2) ;
des trous excentriques (10) sont disposés de manière relative sur les deux pieds en arc de cercle parallèles de la fente en forme de U (4) ;
ladite structure d'arbre comprend un goujon (5) et un manchon d'extension (7), une extrémité du goujon (5) est reliée en rotation entre les trous excentriques (10) sur les deux pieds en arc de cercle parallèles par un boulon excentrique (11), l'autre extrémité du goujon (5) est fixée au manchon d'extension (7) ;
ledit goujon (5) est vissé avec l'écrou de fixation (6).

2. Dispositif de support d'ouvre-bouche selon la revendication 1, **caractérisé en ce que** l'extrémité de la partie verticale de la structure de préhension est formée d'un seul tenant avec la structure excentrique.

3. Dispositif de support d'ouvre-bouche selon la revendication 1, **caractérisé en ce que** l'extrémité de la partie verticale de la structure de préhension est fixée à la structure excentrique, et la partie de liaison de la fente en forme de U (4) reliant les deux pieds en arc de cercle parallèles est fixée à la partie verticale de la structure de préhension par l'intermédiaire d'un verrou (15).

4. Dispositif de support d'ouvre-bouche selon la revendication 3, **caractérisé en ce que** la paroi latérale de la partie verticale de la structure de préhension est pourvue d'une vis de réglage (17) pour régler le mouvement du verrou (15).

5. Dispositif de support d'ouvre-bouche selon la revendication 2 ou 4, **caractérisé en ce qu'**une tige d'extension (19) est fixée dans le manchon d'extension (7) par un boulon (13).

6. Dispositif de support d'ouvre-bouche selon la revendication 5, **caractérisé en ce qu'**une extrémité de la tige d'extension (19) est fixée dans le manchon d'extension et l'autre extrémité est pourvue d'une structure de support stable (20).
